# EUROPEAN PATENT SPECIFICATION

(11) **EP 3 641 568 B1**
(45) Date of publication and mention of the grant of the patent: **28.01.2026**
(21) Application number: 18740308.4
(22) Date of filing: 19.06.2018
(51) Int. Cl.: A23L 33/135, A61K 35/747, A61P 25/16

(54) **COMPOSITION COMPRISING BACTERIAL STRAINS BELONGING TO THE SPECIES LACTOBACILLUS SALIVARIUS FOR THE TREATMENT OF PARKINSON'S DISEASE**
ZUSAMMENSETZUNG MIT BAKTERIENSTÄMMEN DER GATTUNG LACTOBACILLUS SALIVARIUS ZUR BEHANDLUNG VON MORBUS PARKINSON
COMPOSITION COMPRENANT DES SOUCHES BACTÉRIENNES APPARTENANT À L'ESPÈCE LACTOBACILLUS SALIVARIUS POUR LE TRAITEMENT DE LA MALADIE DE PARKINSON

(30) Priority: 19.06.2017 IT 201700068009
(43) Date of publication of application: 29.04.2020
(73) Proprietor: Probiotical S.p.A., 28100 Novara (IT)
(72) Inventor: MOGNA, Giovanni, 28100 Novara (IT)
(74) Representative: Benedetto, Marco
(86) International application number: PCT/IB2018/054509
(87) International publication number: WO 2018/234995

(56) References cited:
- WO-A1-03/010298
- WO-A1-2015/170159
- WO-A1-2017/085655
- WO-A1-2017/208172
- WO-A2-2004/003235
- WO-A2-2006/102536
- WO-A2-2017/025936
- US-A1- 2014 243 384
- DRAGO L ET AL: "Effects of Lactobacillus salivarius LS01 (DSM 22775) treatment on adult atopic dermatitis: A randomized placebo-controlled study", INTERNATIONAL JOURNAL OF IMMUNOPATHOLOGY AND PHARMACOLOGY, vol. 24, no. 4, 2011, SAGE PUBLICATIONS INC. ITA, pages 1037 - 1048, XP002778674, ISSN: 0394-6320
- PEREZ-PARDO PAULA ET AL: "The gut-brain axis in Parkinson's disease: Possibilities for food-based therapies", EUROPEAN JOURNAL OF PHARMACOLOGY, vol. 817, 23 May 2017 (2017-05-23), pages 86 - 95, XP085268480, ISSN: 0014-2999, DOI: 10.1016/J.EJPHAR.2017.05.042
- MAZZINI L ET AL: "Theme 10 Therapeutic Strategies: TST-01 Potential role of gutmicrobiota in ALS pathogenesis", AMYOTROPHIC LATERAL SCLEROSIS AND FRONTOTEMPORAL DEGENERATION -28TH INTERNATIONAL SYMPOSIUM ON ALS/MND, vol. 18, no. Supplement 2, 1 November 2017 (2017-11-01), TAYLOR AND FRANCIS LTD NLD, pages 245 - 260, XP002778675, ISSN: 2167-9223, DOI: 10.1080/21678421.2017.1374612

## Description

The present invention relates to a composition comprising at least one bacterial strain, and/or derivatives thereof, belonging to the species *Lactobacillus salivarius* for the curative and/or preventive treatment of neurodegenerative pathologies, such as Parkinson's disease.

Among neurodegenerative diseases we may mention Parkinson's disease. "Parkinson's disease", also defined as PD, *idiopathic parkinsonism,* primary *parkinsonism,* hypokinetic rigid syndrome or agitated paralysis, refers to a neurodegenerative disease, i.e. a disease caused by a progressive deterioration of brain cells. The pathology is characterised by a slow, progressive loss of one or more functions of the nervous system and manifests itself with typical motor symptoms, such as involuntary and uncontrollable tremors, rigidity, slowness in movement, difficulty in walking and, in the advanced stages, dementia and cognitive and behavioural problems. The typical motor symptoms of the condition are the result of the death of the cells that synthesise and release dopamine. These cells are found in the *substantia nigra*, a region of the mesencephalon.

Gastrointestinal symptoms, including constipation, often precede the motor disorders provoked by Parkinson's disease. Lewy bodies and alpha-synuclein, which are neuropathological markers of PD, seem to make their appearance in the gut earlier than in the brain and the presence of an inflammation of the colon in patients with PD is increasingly evident, and well documented. This evidence has led to the hypothesis that Parkinson's disease begins in the gut and spreads to the brain. There are indications that the microbiota of Parkinson's patients exhibit characteristic alterations and the protein alpha-synuclein, which is one of the causes responsible for cell death in the brain of Parkinson's disease sufferers, is also found in the gut of the same patients.

The aim of the currently available pharmacological therapy for Parkinson's disease is to compensate for the lack of dopamine in the striatum by mimicking physiological stimulation. It is well known that the main treatment consists in the administration of levodopa, which has the function of increasing the concentration of dopamine in the brain. Dopamine is not able to cross the blood-brain barrier, which levodopa, by contrast, can pass through.

The positive effects of levodopa are geared towards the motor symptoms of the pathology, but this drug is often responsible for the onset of severe dyskinesias. That is why there is a tendency to defer treatment with levodopa as long as possible.

Following therapy with levodopa, an individual stricken with Parkinson's disease passes through a first "therapy honeymoon" period, which lasts from 2 to 5 years, in which the therapy almost completely controls the symptoms and the individual enjoys an almost normal life. In fact, the drug is effective in any individual with Parkinson's disease, irrespective of the duration, severity and age of onset of the disease. Subsequently, however, there is a stage in which the effectiveness of levodopa decreases and as a consequence there is a worsening of the symptoms of the disease.

Unfortunately, still today the therapeutic remedies for Parkinson's disease are symptomatic and are not able to bring about a remission of the disease.

It has also been demonstrated that some dopamine agonists seem to have neuroprotective properties; practically speaking, they seem to slow down the progression of the neurodegeneration, without, however, removing the causes of the disease. In fact, dopamine agonist drugs show a moderate effectiveness and slow the motor symptoms, but they cause side effects such as gastrointestinal and cardiovascular disorders, fibrosis, drowsiness and, compared to levodopa, a higher frequency of psychiatric problems. It has in fact been observed that the use of such drugs seems to be correlated to impulse control disorders, such as, for example, pathological gambling, hypersexuality and *binge eating disorder,* which manifest themselves in 13-17% of the patients who use this therapy. For this reason the treatment starts with low doses, subsequently passing to higher doses in a gradual manner.

At present, there is no available composition for the preventive or curative treatment of Parkinson's disease, and the symptoms and disorders associated therewith, which is free of serious side effects and has long-lasting effectiveness.

WO2004/003235 discloses probiotic strains of *L.salivarius,* which are used for treating neurodegenerative diseases, such as Parkinson's disease.

One aim of the present invention is to provide a composition for the preventive or curative treatment of Parkinson's disease, and the symptoms and disorders associated therewith, which is effective, substantially free of side effects and well tolerated also in the case of prolonged administration and also in human subjects affected by other pathologies.

In response to said need, the present invention provides a composition for use according to the appended claims.

The subject matter of the present invention relates to a composition comprising an effective amount of a mixture which comprises or, alternatively, consists of at least one probiotic bacterial strain, and/or derivatives thereof, belonging to the species *Lactobacillus salivarius,* which is *Lactobacillus salivarius* LS03 DSM 22776, for use in a method for the curative and/or preventive treatment of at least one neurodegenerative pathology, or at least one symptom or disorder associated therewith, in a human subject, wherein said treatment comprises the administration of said composition to the subject and wherein said at least one neurodegenerative pathology is Parkinson's disease.

The subject matter of the present invention further relates to a method for the curative and/or preventive treatment of at least one neurodegenerative pathology, or at least one symptom or disorder associated therewith, in a human subject, wherein said at least one neurodegenerative pathology is Parkinson's disease and wherein said method of treatment comprises the administration of a composition comprising or, alternatively, consisting of an effective amount of a mixture which comprises or, alternatively, consists of at least one probiotic bacterial strain, and/or derivatives thereof, belonging to the species *Lactobacillus salivarius* as described in detail in the present invention, the at least one probiotic strain comprising L. salivarius LS03 DSM 22776.

Preferred embodiments of the present invention will emerge clearly from the detailed description that follows and are specified in the appended claims.

In the accompanying Figures, the bacterial strains are indicated by codes with reference to Table 1.
Figure 1 shows the effect of the bacterial strains in Table 1 on the production of the superoxide anion for the evaluation of oxidative stress in subjects affected by Parkinson's disease.
Figure 2 shows the data of Figure 1 for female human subjects.
Figure 3 shows the data of Figure 1 for male human subjects.
Figure 4 relates to the activity of modulation of the bacterial strains on the Th1 (pro-inflammatory response)/Th2 (anti-inflammatory response) ratio in subjects affected by Parkinson's disease. A decrease in the Th1/Th2 ratio indicates a modulation in an anti-inflammatory direction.
Figure 5 shows the data of Figure 4 for female human subjects.
Figure 6 shows the data of Figure 4 for male human subjects.
Figure 7 shows the analysis of the results and the selection of the strains with a greater differentiated effect on the production of the superoxide anion for the evaluation of oxidative stress and on the anti-inflammatory effect for both male human subjects and female human subjects.
Figures 8 and 9 show the reduction in Cytochrome C, which is directly proportional to the production of the superoxide anion (O₂-), an indicator of oxidative stress, respectively in healthy subjects and subjects affected by Parkinson's disease.
Figure 10 shows the evaluation of the protection of membrane integrity (Trans-Epithelial Electrical Resistance, TEER) after 2 hours of stimulation with pro-inflammatory agents.
Figure 11 shows the evaluation of the restoration of membrane integrity (Trans-Epithelial Electrical Resistance, TEER) after 2 hours of stimulation with pro-inflammatory agents.
Figure 12 shows the evaluation of the Th1/Th2 ratio obtained from the values of the release of the cytokines TNF-alpha and IL-10 *in vitro* on PBMCs (Peripheral Blood Mononuclear Cells) isolated from the blood of Parkinson's patients stimulated for 24 hours with probiotic strains.
Figure 13 shows the inhibition of the release of cytokines IL-88 *in vitro* on PBMCs (Peripheral Blood Mononuclear Cells) isolated from the blood of Parkinson's patients stimulated for 24 hours with the probiotic strains.
Figure 14 relates to the inhibition of the release of the pro-inflammatory cytokine IL-17 by probiotic bacterial strains in healthy human subjects.
Figure 15 relates to the inhibition of the release of the pro-inflammatory cytokine IL-17 by probiotic bacterial strains in human subjects with PD.

Probiotics are a broad and heterogeneous group of microorganisms that provide a beneficial effect on human health. In fact, a number of studies have demonstrated a growing interest in the use of probiotics, in addition to conventional anti-inflammatory therapies, in order to exploit their particular properties.

In fact, it has been amply demonstrated that probiotics have an anti-inflammatory effect, that, by modulating the release of pro-inflammatory cytokines, they are capable of having a direct action in inhibiting the growth of pathogenic agents and that they are capable of colonising and protecting the intestinal membrane.

Following extensive experimentation, the inventors have developed a composition, comprising at least one probiotic bacterial strain and/or derivatives thereof, effective for the curative and/or preventive treatment of Parkinson's disease, and analogous neurodegenerative pathologies, which is well tolerated and essentially free of the connected side effects reported in relation to the presently available pharmacological therapies.

In the context of the present invention, "bacterial strains" and/or the "derivatives" thereof are claimed.

"Bacterial strains" is meant to include: a) viable bacterial strains and b) dead bacterial strains. The group of dead bacterial strains (b) is meant to include, by way of non-limiting example: b1) bacterial cell wall extracts, b2) tyndallized bacteria, b3) sonicated bacteria (bacteria treated with a sonication process), and b4) lysed bacteria.

"Derivatives" is meant to include, by way of non-limiting example, bioactive peptides derived from a bacterial strain (bacterial metabolites).

In particular, it has been found that the strain *Lactobacillus salivarius* LS03 ((ID1382) - DSM 22776 deposited by Probiotical S.p.A. (Italy) with the DSMZ on 23.07.2009) and the derivatives thereof perform important anti-inflammatory activities in subjects suffering from Parkinson's disease both in opposing the progress of oxidative stress and in inhibiting the release of pro-inflammatory cytokines.

Moreover, said bacterial strain *Lactobacillus salivarius* LS03, and the derivatives thereof, has demonstrated to be an excellent strain to be used in both the prevention and repair of damage to the intestinal membrane.

Specifically, *Lactobacillus salivarius* LS03 and the derivatives thereof are effective in significantly decreasing oxidative stress, significantly maintaining and restoring membrane permeability and in significantly modulating the most important cytokine patterns.

"Neurodegenerative disease" means a pathology of the central or peripheral nervous system, characterised by a chronic, selective process of cell death or severe degeneration affecting neurons. Non-limiting examples of neurodegenerative diseases are Alzheimer's disease, Parkinson's disease, Amyotrophic Lateral Sclerosis (ALS) or Lou Gehrig's disease, senile dementia, Huntington's disease, progressive supranuclear palsy, frontotemporal dementia and Lewy body dementia.

Within the scope of the present invention, "treatment" of neurodegenerative diseases means therapy aimed at restoring the health conditions of a subject, maintaining the existing conditions and/or preventing a rapid worsening, or slowing down the worsening, of said health conditions.

Within the scope of the present invention, "prevention" of pathologies means a therapy aimed at preventing the occurrence of such a pathology in a subject, also, but not only, as a complication or effect of a pathological condition or pre-existing disorder.

In general and outside the scope of the claimed invention, the at least one probiotic bacterial strain, and/or derivatives thereof, is preferably selected in the group comprising or, alternatively, consisting of: the strain *Lactobacillus salivarius* LS03 (ID1382) - DSM 22776 (deposited by Probiotical S.p.A. with the DSMZ on 23.07.2009), the strain LS01 DSM 22775 (deposited by Probiotical S.p.A. with the DSMZ on 23.07.2009), the strain LS02 DSM 32204 - (deposited by Probiotical S.p.A. with the DSMZ on 13.11.2015), the strain LS 06 DSM 26037 (deposited by Probiotical S.p.A. with the DSMZ on 06.06.2012), the strain LS 07 DSM 29476 (deposited by Probiotical S.p.A. with the DSMZ on 09.10.2014) and mixtures thereof; more preferably said at least one strain is LS03 DSM 22776, LS01 DSM 22775 or mixtures thereof.

In the composition according to the present invention, said mixture preferably further comprises at least one probiotic bacterial strain, and/or derivatives thereof, selected in the group comprising or, alternatively, consisting of:
*Lactobacillus plantarum* LP01 LMG P-21021 (deposited by Mofin Srl with the BCCM-LMG on 16.10.2001), *Lactobacillus reuteri* DLLRE 09 DSM 25685 (deposited by Probiotical S.p.A. with the DSMZ on 16.02.2012), *Lactobacillus acidophilus* LA 02 DSM 21717 (deposited by Probiotical S.p.A. with the DSMZ on 06.08.2008), *Lactobacillus rhamnosus* LR 06 DSM 21981 (deposited by Probiotical S.p.A. with the DSMZ on 14.11.2008) if the subject is male (of the male gender);
or, if the subject is female (of the female gender), at least one strain, and/or derivatives thereof, selected from among *Bifidobacterium lactis* BS01 LMG P-21384 (deposited by Anidral S.r.l. with the BCCM LMG on 31.01.2002) and a mixture (DLBL5S), preferably in a ratio of 1:1:1:1:1, consisting of *Bifidobacterium longum* DLBL 07 DSM 25669 (deposited by Probiotical S.p.A. with the DSMZ on 16.02.2012), *Bifidobacterium longum* DLBL 08 DSM 25670 (deposited by Probiotical S.p.A. with the DSMZ on *16.02.2012), Bifidobacterium longum* DLBL 09 DSM 25671 (deposited by Probiotical S.p.A. with the DSMZ on 16.02.2012), *Bifidobacterium longum* DLBL 10 DSM 25672 (deposited by Probiotical S.p.A. with the DSMZ on 16.02.2012), *Bifidobacterium longum* DLBL 11 DSM 25673 (deposited by Probiotical S.p.A. with the DSMZ on 16.02.2012).

In a preferred embodiment of the composition according to the present invention, said mixture comprises the strain *Lactobacillus salivarius* LS03 (ID1382) - DSM 22776, and also at least one probiotic bacterial strain, and/or derivatives thereof, selected in the group comprising or, alternatively, consisting of:
*Lactobacillus plantarum* LP01 LMG P-21021 (deposited by Mofin Srl with the BCCM-LMG on 16.10.2001), *Lactobacillus reuteri* DLLRE 09 DSM 25685 (deposited by Probiotical S.p.A. with the DSMZ on 16.02.2012), *Lactobacillus acidophilus* LA 02 DSM 21717 (deposited by Probiotical S.p.A. with the DSMZ on 06.08.2008), *Lactobacillus rhamnosus* LR 06 DSM 21981 (deposited by Probiotical S.p.A. with the DSMZ on 14.11.2008) if the subject is male (of the male gender) ;
or, if the subject is female, at least one strain, and/or the derivatives thereof, selected from among *Bifidobacterium lactis* BS01 LMG P-21384 (deposited by Anidral S.r.l. with the BCCM LMG on 31.01.2002) and a mixture (DLBL5S), preferably in a ratio of 1:1:1:1:1, consisting of *Bifidobacterium longum* DLBL 07 DSM 25669 (deposited by Probiotical S.p.A. with the DSMZ on 16.02.2012), *Bifidobacterium longum* DLBL 08 DSM 25670 (deposited by Probiotical S.p.A. with the DSMZ on 16.02.2012), *Bifidobacterium longum* DLBL 09 DSM 25671 (deposited by Probiotical S.p.A. with the DSMZ on 16.02.2012), *Bifidobacterium longum* DLBL 10 DSM 25672 (deposited by Probiotical S.p.A. with the DSMZ on 16.02.2012), *Bifidobacterium longum* DLBL 11 DSM 25673 (deposited by Probiotical S.p.A. with the DSMZ on 16.02.2012).

In a further embodiment of the composition according to the present invention, said mixture comprises the strain *Lactobacillus salivarius* LS03 (ID1382) - DSM 22776, the strain LS01 DSM 22775 and also at least one probiotic bacterial strain, and/or derivatives thereof, selected in the group comprising or, alternatively, consisting of:
*Lactobacillus plantarum* LP01 LMG P-21021 (deposited by Mofin Srl with the BCCM-LMG on 16.10.2001), *Lactobacillus reuteri* DLLRE 09 DSM 25685 (deposited by Probiotical S.p.A. with the DSMZ on 16.02.2012), *Lactobacillus acidophilus* LA 02 DSM 21717 (deposited by Probiotical S.p.A. with the DSMZ on 06.08.2008), *Lactobacillus rhamnosus* LR 06 DSM 21981 (deposited by Probiotical S.p.A. with the DSMZ on 14.11.2008) if the subject is male (of the male gender);
or, if the subject is female (of the female gender), at least one strain, and/or derivatives thereof, selected from among *Bifidobacterium lactis* BS01 LMG P-21384 (deposited by Anidral S.r.l. with the BCCM LMG on 31.01.2002) and a mixture (DLBL5S), preferably in a ratio of 1:1:1:1:1, consisting of *Bifidobacterium longum* DLBL 07 DSM 25669 (deposited by Probiotical S.p.A. with the DSMZ on 16.02.2012), *Bifidobacterium longum* DLBL 08 DSM 25670 (deposited by Probiotical S.p.A. with the DSMZ on 16.02.2012), *Bifidobacterium longum* DLBL 09 DSM 25671 (deposited by Probiotical S.p.A. with the DSMZ on 16.02.2012), *Bifidobacterium longum* DLBL 10 DSM 25672 (deposited by Probiotical S.p.A. with the DSMZ on 16.02.2012), *Bifidobacterium longum* DLBL 11 DSM 25673 (deposited by Probiotical S.p.A. with the DSMZ on 16.02.2012).

The composition according to the present invention can be for use in human subjects or for veterinary use, for example, but without limitation, in pets such as cats or dogs, or in other mammals. The composition according to the present invention is preferably for use in humans.

In one embodiment, the administration of the composition to the subject takes place orally, for example in the form of a pill, tablet, which may also be coated, a capsule, solution, suspension, syrup, food containing the probiotic bacteria, or in any other form known to the person skilled in the art.

It remains understood that, if the treatment according to the invention comprises the administration of more than one bacterial strain and/or derivatives thereof, said administration according to the invention can take place simultaneously, for example in a single formulation, or in a rapid sequence, for example with two or more formulations taken by the subject in any order, in a sequence closely spaced in time (e.g. within 1 to 30 minutes) in two distinct compositions.

The composition for use according to the present invention can comprise, in addition to a bacterial strain of the species *Lactobacillus salivarius* and/or derivatives thereof, at least one inert ingredient, such as at least one excipient among the ones commonly used and known to the person skilled in the art.

"Inert ingredient" means any substance, or combination of substances, auxiliary to the production of a pharmaceutical, dietary or nutraceutical form, which is to be found in the finished product and is not the active ingredient, although it can modify the stability, release or other characteristics thereof.

Non-limiting examples of such ingredients, as is known to the person skilled in the art of formulations in the pharmaceutical, nutraceutical or food industry, are excipients such as diluents, absorbents, lubricants, colourants, surfactants, antioxidants, sweeteners, binders, disaggregating agents and the like.

In one embodiment, the composition for use according to the present invention comprises, in addition to one or more bacterial strains and/or derivatives thereof, at least one further active ingredient of natural or synthetic origin. Non-limiting examples of said compounds are vitamins, antioxidants, or vegetable substances and preparations (botanicals).

The composition can be in the form of a medical device (in accordance with the European medical device regulation (EU) 2017/745 - (MDR) and Directive 93/42/EEC -(MDD)), or a composition suitable for food or nutraceutical use, for example as a dietary supplement, or in the form of a pharmaceutical composition.

The pharmaceutical composition, dietary supplement or medical device of the present invention can be solid, liquid or semisolid, for example as a suspension or gel, and can be in any form known the person skilled in the art of the food, pharmaceutical or nutraceutical formulations, such as, by way of non-limiting example, in the form of a capsule, tablet, or powder that is at least partially dissolvable in the mouth or water soluble, granules, pellets or microparticles optionally contained in a sachet or in a capsule or mini-tablet, a liquid or semisolid preparation, gel, suspension, solution, two-phase liquid system and equivalent forms.

The following experimental part provides examples of practical embodiments of the invention.

Parts of the examples that may involve subject matter the is not part of the claimed invention should be considered as illustrative and/or comparative thereto.

### EXPERIMENTAL PART

The present *in vitro* study, conducted in humans (*in vivo*) by the inventors, aimed to analyse the activity of different bacterial strains on PBMCs (Peripheral Blood Mononuclear Cells) isolated from the blood of patients affected by Parkinson's disease in order to assess the direct effects of the microorganisms on oxidative stress and on the release of the cytokines most involved in the pathology.

The inventors conducted the present *in vitro* study particularly in order to analyse the effects of the bacterial strain *Lactobacillus salivarius* LS03 (DSM 22776) vis-à-vis the other selected bacterial strains. The comparison was conducted on the main cellular subpopulations involved in the innate and acquired immune responses. Said subpopulations were isolated from the peripheral blood of subjects with Parkinson's disease, and were compared to those of healthy subjects.

**Table 1**

| **STUDY STRAINS** | **Number - and deposit date** | **pro-Th2** | **anti-IL17A** |
|---|---|---|---|
| *Lactobacillus acidophilus* LA 02 | DSM 21717 - 06.08.2008 | X | X |
| *L. delbrueckii subsp delbrueckii* LDD01 | DSM 22106 - 10.12.2008 | X | X |
| *Lactobacillus plantarum* LP01 | LMG P-21021 - 16.10.2001 | X | X |
| *Lactobacillus plantarum* LP02 | LMG P-21020 - 16.10.2001 | X | |
| *Lactobacillus rhamnosus* LR 06 | DSM 21981 - 14.11.2008 | X | |
| *Lactobacillus reuteri* LRE04 | DSM 23880 - 05.08.2010 | X | X |
| *Lactobacillus reuteri* DLLRE 09 | DSM 25685 - 16.02.2012 | X | X |
| *Lactobacillus salivarius* LS01 | DSM 22775 - 23.07.2009 | X | X |
| *Lactobacillus salivarius* LS02 | DSM 32204 - 13.11.2015 | X | X |
| *Lactobacillus salivarius* LS03 | DSM 22776 - 23.07.2009 | X | X |
| *Lactobacillus salivarius* LS 06 | DSM 26037 - 06.06.2012 | X | X |
| *Lactobacillus salivarius* LS 07 | DSM 29476 - 09.10.2014 | X | |
| GG | 1736 | X | |
| *Bifidobacterium breve* BR 03 | DSM 16604 20.07.2004 | X | |
| *Bifidobacterium breve* BR 05 | DSM 29494 09.10.2014 | X | |
| *Bifidobacterium breve* BS01 | LMG P-21384 31.01.2002 | X | |
| DLBL mixture of 5 strains (DLBL5S) | *Bifidobacterium longum* DLBL 07 DSM 25669, *Bifidobacterium longum* DLBL 08 DSM 25670, *Bifidobacterium longum* DLBL 09 DSM 25671, *Bifidobacterium longum* DLBL 10 DSM 25672, *Bifidobacterium longum* DLBL 11 DSM 25673, all deposited on 16.02.2012 | X | |

Assessment of the implications of the action of the microorganisms on oxidative stress and on the modulation of the cytokine patterns in an *in vitro* model of PBMCs isolated from patients with Parkinson's disease.

### MATERIALS and METHODS

### Strains: see Table 1

Subjects affected by Parkinson's disease (PD):
- 15 females and 25 males;
- mean age: 69.9±8 years;
- 27 patients undergoing pharmacological treatment with Levodopa (17 males/10 females);
- 13 patients without pharmacological treatment (8 males/ 5 females);
- mean duration of the disease: 5±4 years; a 20 cc sample of venous blood was taken from all subjects;
- the blood was mixed with heparin, as an anticoagulant, and processed on the same day it was collected.

The Peripheral Blood Mononuclear Cells (PBMCs) were isolated from the peripheral blood of subjects suffering from Parkinson's disease using the standard protocol of dextran sedimentation and Histopaque gradient centrifugation.

In this study, use was made of all the bacterial strains of the collection of Probiotical S.p.a. in Table 1, which were cultured at T = 37°C in the selective medium MRS (De Man, Rogosa and Sharpe medium) for Lactobacilli and MRS + 0.05% cysteine (by weight/total weight) for Bifidobacteria.

In the cellular model of PBMCs, the *in vitro* effects of the microorganisms on the production of the oxygen free radicals were assessed by means of an indirect spectrophotometric technique, the Superoxide Anion Assay (ROS test).

The modulation of the *in vitro* release of the most important pro- and anti-inflammatory cytokines by the PBMCs after incubation with the different bacterial strains under examination was also investigated, and the (Th1/Th2) ratio between the two cytokine families was subsequently assessed.

The data analysis and statistical assessment were performed with the ANOVA test; the data were considered significant for values of p<0.05.

### RESULTS

### Evaluation of oxidative stress

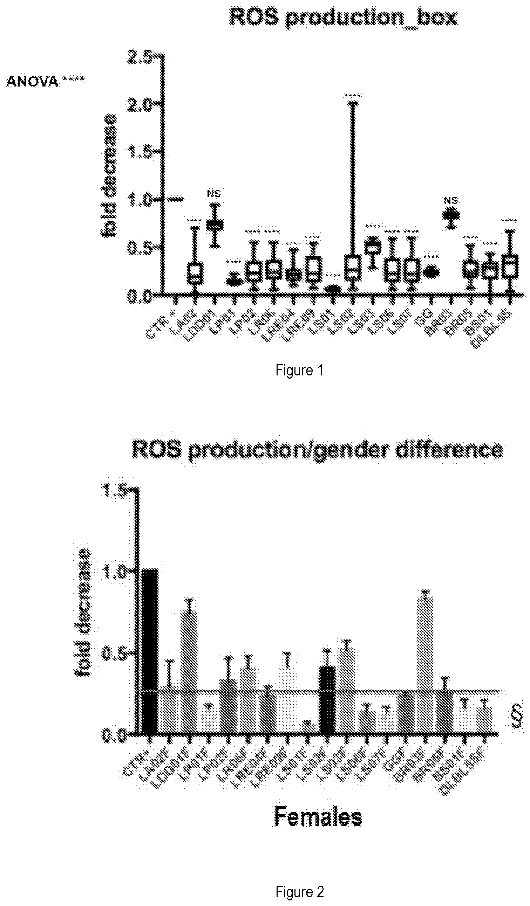

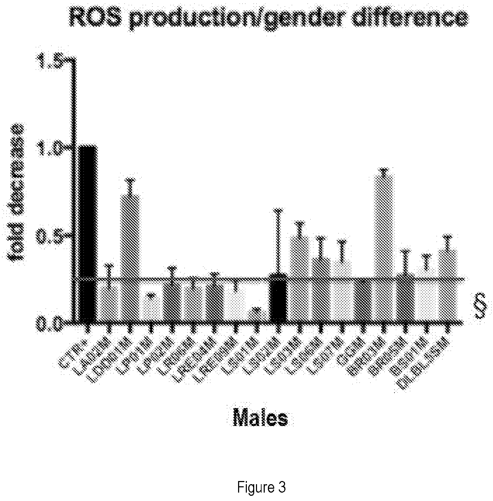

The monocytes (1×10⁶ cells/well) of Parkinson's patients were treated for 24 hours with the bacterial strains in Table 1. An indirect spectrophotometric test was then used to assess the reduction in Cytochrome C, which is directly proportional to the production of the superoxide anion (O₂-), an indicator of oxidative stress (Figure 1). Figures 2 and 3 (§ = indicator of very high activity) show the difference in the activity of the different bacterial strains tested on PBMCs originating from male and female patients.

### Evaluation of the release of the cytokines Th1 (pro-inflammatory) and Th2 (anti-inflammatory)

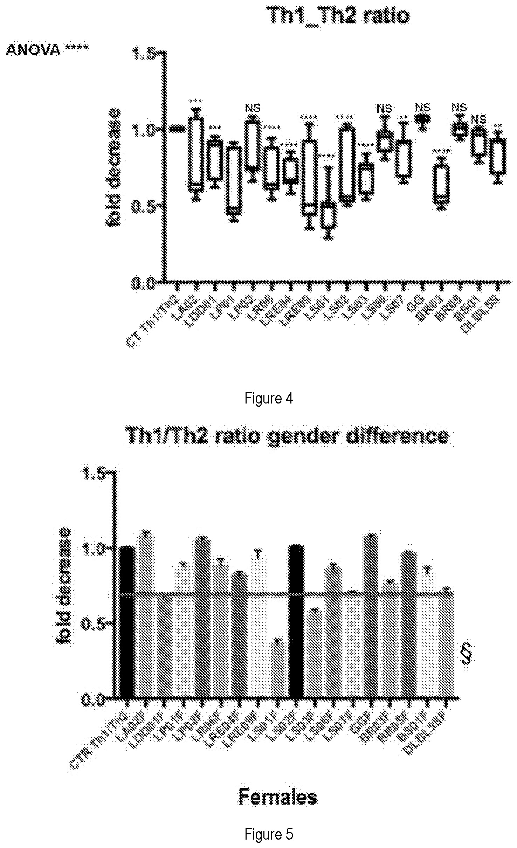

The PBMCs isolated from the blood of Parkinson's patients were stimulated for 24 hours with the probiotic strains (incubation at 37°C and 5% CO₂). The release of the pro-inflammatory cytokine TNF-alpha (pro-Th1) and of the anti-inflammatory cytokine IL-10 (pro-Th2) was assessed. The release values obtained were used to calculate a Th1/Th2 ratio which indicated the activity of the strains on pro- or anti-inflammatory modulation (Figure 4, ANOVA Test p<0.0001****, NS = not significant).

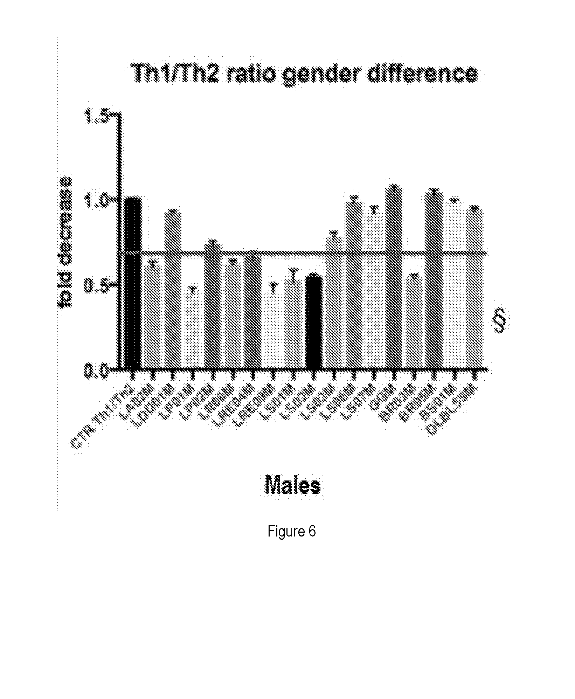

Figures 5 and 6 (§ = indicator of very high activity) show the difference in the activity of the different microorganisms on PBMCs originating from male patients and female patients.

Conclusions: 12 bacterial strains out of 17 modulate the release of pro- and anti-inflammatory cytokines in a significant manner.

### Statistical analysis of the results

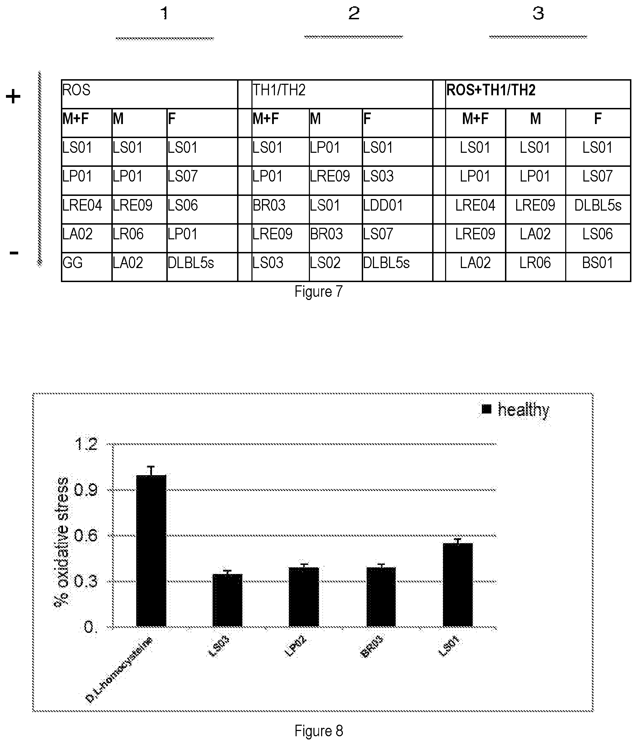

In the Table in Figure 7, the strains are listed in order from the "strongest" to the "weakest" (direction of the arrow); the statistical analysis (regression) was corrected for age parameters, duration of disease, UPDRS (Unified Parkinson's Disease Rating Scale) and treatment with Levodopa. The bacterial strains tested for males (M) and females (F) (together and separate) were selected:
- for their activity on the production of ROS (part 1 of the table in Figure 7);
- for their activity on cytokines (TH) (part 2 of the table in Figure 7).

Combining the overall distance both of ROS and TH from neutrality (ROS=1 and TH=1), the tested bacterial strains with a greater effect on both activities were then selected (part 3 of the table).

Further studies were then carried out on subjects affected by Parkinson's disease (15 females and 25 males) and on healthy subjects.

The intestinal permeability of the strains was also determined.

### Oxidative stress in healthy subjects compared with patients affected by Parkinson's disease.

The strains were cultured at T = 37°C in the selective medium MRS (De Man, Rogosa and Sharpe medium) for Lactobacilli and MRS + 0.05% cysteine (by weight/total weight) for Bifidobacteria. The PBMCs (Peripheral Blood Mononuclear Cells), cellular model of the immune system, were isolated from heparinised venous blood of healthy donors and PD patients using standard dextran sedimentation and Histopaque gradient centrifugation; ELISA (Enzyme-Linked Immunoabsorbent Assay) tests were conducted on these cells in order to evaluate the cytokine patterns. The monocytes were isolated from PBMCs of both healthy donors and PD patients by adhesion after 24 hours in 12-well plates; the superoxide anion assay (ROS test) was conducted on them to assess oxidative stress.

### Evaluation of oxidative stress

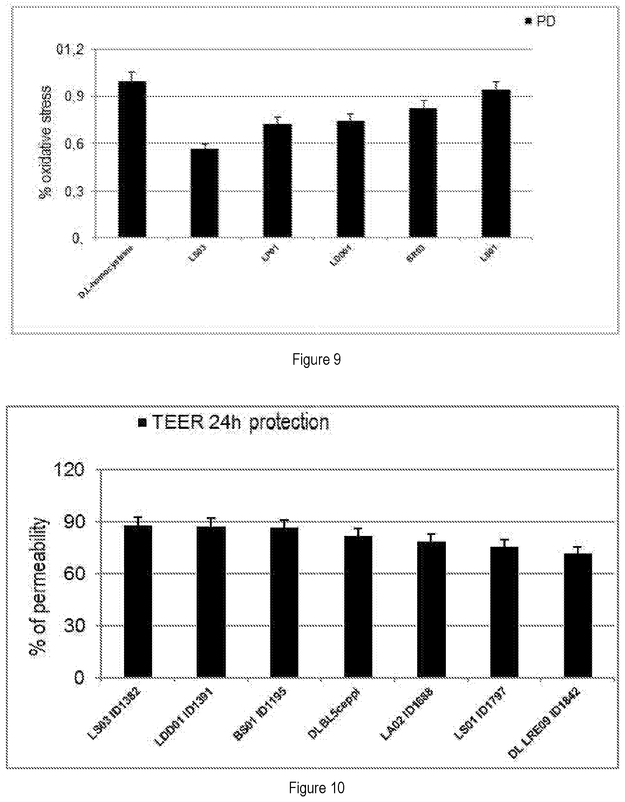

The monocytes (1×10⁶ cells/well) of healthy subjects (10 males and 10 females) and Parkinson's patients (25 males, 15 females) were treated for 24 hours with the probiotic strains specified in Figures 8 and 9, i.e. LS03, LP02, BR03, LS01 for healthy subjects and LS03, LP01, LDD01, BR03 and LS01 for Parkinson's disease patients, then stimulated with 5 mM of homocysteine in RPMI 1640 medium for another 2 hours. The reduction in Cytochrome C, which is directly proportional to the production of the superoxide anion (O₂-), an indicator of oxidative stress, was then assessed by means of an indirect spectrophotometric test.

### Evaluation of Intestinal Permeability

### MATERIALS and METHODS

CACO-2 cells, a model of intestinal epithelial cells, were cultured in 24-well Transwell plates, in DMEM medium (Gibco); a test was conducted on one of them for the purpose of evaluating the integrity of the intestinal membrane by measuring the Trans-Epithelial Electrical Resistance (TEER).

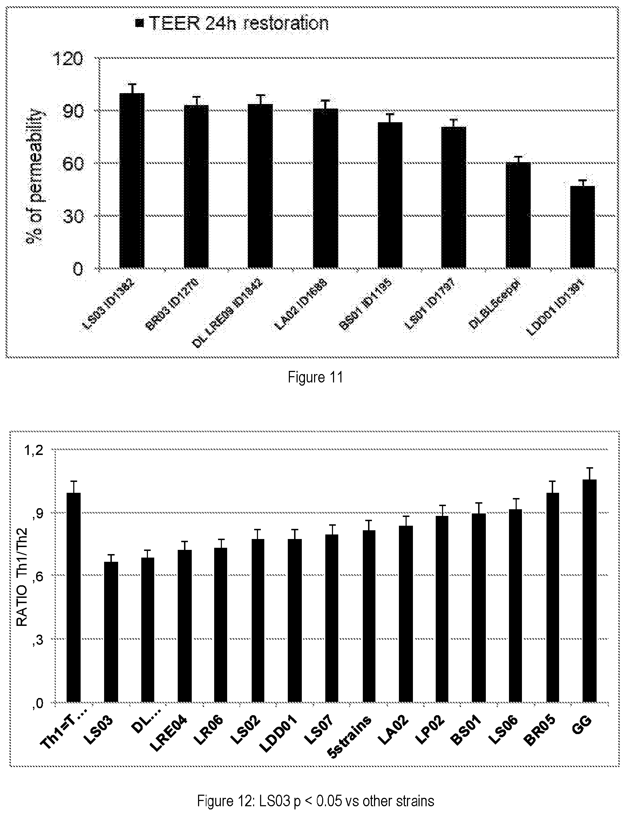

All of the strains specified in Figures 10 and 11 were tested on a 24-multiwell plate of CACO-2 cells grown to confluence, *in vitro* model of intestinal epithelial tissue. Two parallel experimental protocols were implemented: a protocol for evaluating protection and one for evaluating the restoration of intestinal membrane integrity.

In both cases, after 2 hours of stimulation with the pro-inflammatory agents and the strains, the supernatant was recovered. 24 hours after replacement of the medium, an assessment was made of the Trans-Epithelial Electrical Resistance (TEER), a recognised parameter of membrane integrity (Figure 10, relating to protection, and Figure 11, relating to restoration).

### Evaluation of the release of cytokines: TNF-alpha and IL-10

The PBMCs isolated from the blood of Parkinson's patients (25 males, 15 females) were stimulated for 24 hours with the probiotic strains specified in Figure 12. The release of the pro-inflammatory cytokine TNF-alpha (pro-Th1) and of the anti-inflammatory cytokine IL-10 (pro-Th2) was evaluated. The release values obtained were used to calculate a Th1/Th2 ratio indicating the activity of the strains on the pro- or anti-inflammatory modulation (Figure 12). Th1= Th2 (ratio = 1) Th1/Th2 = 1 indicates a system in equilibrium. Th1/Th2 <1 indicates an anti-inflammatory activity.

### Evaluation of the release of the cytokine IL-8

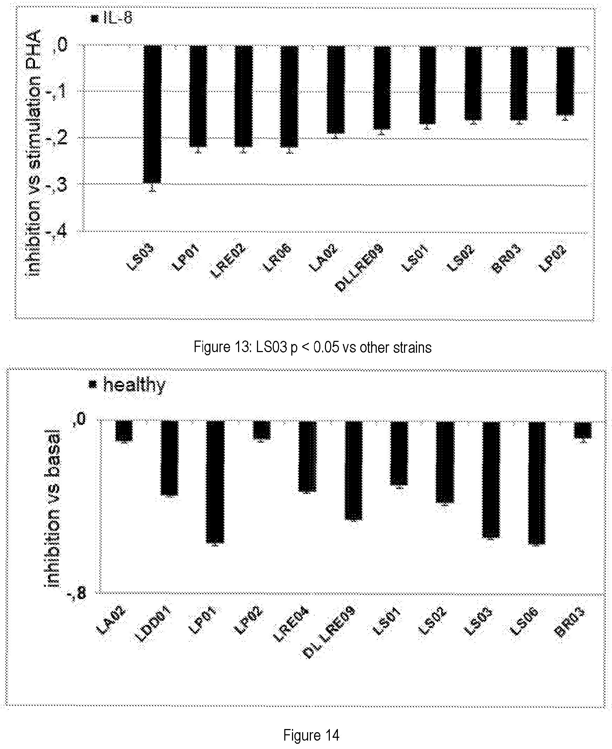

The PBMCs isolated from the blood of healthy donors (10 males, 10 females) stimulated with phytohaemagglutinin (PHA) (positive control) were incubated for 24 hours with the bacterial strains specified in Figure 13. An assessment was made of the release of the pro-inflammatory cytokine IL-8, an important cytokine associated with inflammation and increased by oxidative stress (Figure 13).

### Evaluation of the release of the cytokine IL-17

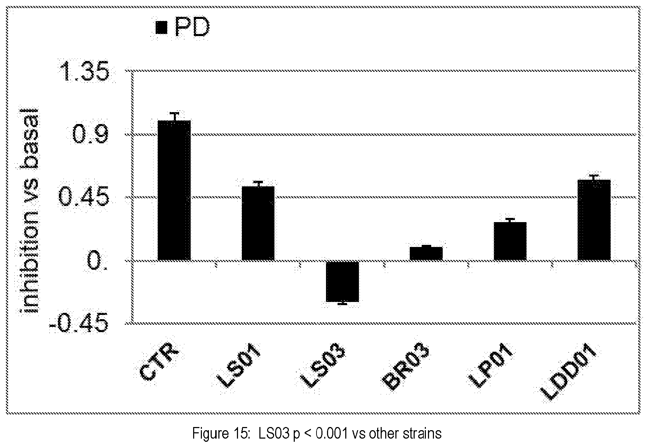

The PBMCs isolated from the blood of healthy donors and Parkinson's patients were incubated for 24 hours with the probiotic strains specified in Figure 14 (healthy subjects) and in Figure 15 (subjects affected by Parkinson's disease). An assessment was made of the release of the pro-inflammatory cytokine IL-17A, a key cytokine in the defence against pathogens, as it is involved in the recruitment and activation of neutrophils.

As may be seen from Figures 14 and 15, the bacterial strain LS03 inhibits the cytokine IL-17A by 50% in healthy subjects and leads to a total inhibition of the cytokine in Parkinson's subjects.

### Conclusions

**(I)** In the *in vitro* model of oxidative stress reproduced in monocytes of healthy subjects and Parkinson's disease sufferers, the bacterial strain LS03 reduced the inflammatory state induced by homocysteine (=1) by 65%, proving to be better than the strains: LP01, LP02, LDD01, BR03 and LS01.
**(II)** In a CACO-2 *in vitro* cellular model of intestinal permeability, the bacterial strain LS03 was capable of maintaining 88% membrane integrity against the inflammatory insult and had a 90% action of restoring membrane integrity.
**(III)** The bacterial strain LS03 further demonstrated to have a considerable activity in modulating the cytokine patterns in an anti-inflammatory direction (Th1<Th2) in Parkinson subjects, and in inhibiting the cytokine IL-8 in healthy subjects and the cytokine IL-17A in a cellular model of PBMCs of healthy subjects and Parkinson's patients.

## Claims

1. A composition comprising an effective amount of a mixture which comprises or, alternatively, consists of at least one bacterial strain belonging to the species *Lactobacillus salivarius,* wherein said at least one bacterial strain, belonging to the species *Lactobacillus salivarius* is *Lactobacillus salivarius* LS03 DSM 22776; said composition being for use in a method for the curative and/or preventive treatment of at least one neurodegenerative pathology, or at least one symptom or disorder associated therewith, in a human subject, wherein said method of treatment comprises the administration of said composition to a human subject and wherein said at least one neurodegenerative pathology is Parkinson's disease.

2. The composition for use according to claim 1, wherein said mixture further comprises at least one bacterial strain, and/or derivatives thereof, selected in the group comprising or, alternatively, consisting of:
*Lactobacillus plantarum* LP01 LMG P-21021 deposited by Mofin Srl with the BCCM-LMG on 16.10.2001, *Lactobacillus reuteri* DLLRE 09 DSM 25685 deposited by Probiotical S.p.A. with the DSMZ on 16.02.2012, *Lactobacillus acidophilus* LA 02 DSM 21717 deposited by Probiotical S.p.A. with the DSMZ on 06.08.2008, *Lactobacillus rhamnosus* LR 06 DSM 21981 deposited by Probiotical S.p.A. with the DSMZ on 14.11.2008 and mixtures thereof, if the human subject is a male subject; or, if the human subject is a female subject, said at least one strain, and/or derivatives thereof, is selected in the group comprising or, alternatively, consisting of:
*Bifidobacterium lactis* BS01 LMG P-21384 deposited by Anidral S.r.l. with the BCCM LMG on 31.01.2002, a mixture DLBL5S, preferably in a ratio of 1:1:1:1:1 , consisting of *Bifidobacterium longum* DLBL 07 DSM 25669 deposited by Probiotical S.p.A. with the DSMZ on 16.02.2012, *Bifidobacterium longum* DLBL 08 DSM 25670 deposited by Probiotical S.p.A. with the DSMZ on 16.02.2012, *Bifidobacterium longum* DLBL 09 DSM 25671 deposited by Probiotical S.p.A. with the DSMZ on 16.02.2012, *Bifidobacterium longum* DLBL 10 DSM 25672 deposited by Probiotical S.p.A. with the DSMZ on 16.02.2012 and *Bifidobacterium longum* DLBL 11 DSM 25673 deposited by Probiotical S.p.A. with the DSMZ on 16.02.2012, and mixtures thereof.

3. The composition for use according to claim 1 or 2, wherein said mixture further comprises *Lactobacillus salivarius* LS01 DSM 22775.

4. The composition for use according to at least one of the preceding claims, wherein said administration takes place orally.

5. The composition for use according to any one of the preceding claims, wherein said method of treatment comprises the administration of said composition to a human subject affected by Parkinson's disease or at risk of contracting Parkinson's disease.

## Patentansprüche

1. Zusammensetzung mit einer wirksamen Menge einer Mischung, die mindestens einen Bakterienstamm der Gattung *Lactobacillus salivarius* umfasst oder alternativ daraus besteht, wobei der mindestens eine Bakterienstamm der Gattung *Lactobacillus salivarius Lactobacillus salivarius* LS03 DSM 22776 ist; wobei die Zusammensetzung zur Verwendung in einem Verfahren zur kurativen und/oder präventiven Behandlung mindestens einer neurodegenerativen Pathologie oder mindestens eines damit verbundenen Symptoms oder einer damit verbundenen Störung bei einem menschlichen Subjekt bestimmt ist, wobei das Behandlungsverfahren die Verabreichung der Zusammensetzung an ein menschliches Subjekt umfasst und wobei es sich bei der mindestens einer neurodegenerativen Pathologie um Morbus Parkinson handelt.

2. Zusammensetzung zur Verwendung nach Anspruch 1, wobei die Mischung ferner mindestens einen Bakterienstamm und/oder Derivate davon umfasst, ausgewählt aus der Gruppe, die umfasst oder alternativ besteht aus:
*Lactobacillus plantarum* LP01 LMG P-21021, hinterlegt von Mofin Srl beim BCCM-LMG am 16.10.2001, *Lactobacillus reuteri* DLLRE 09 DSM 25685, hinterlegt von Probiotical S.p.A. beim DSMZ am 16.02.2012, *Lactobacillus acidophilus* LA 02 DSM 21717, hinterlegt von Probiotical S.p.A. beim DSMZ am 06.08.2008, *Lactobacillus rhamnosus* LR 06 DSM 21981, hinterlegt von Probiotical S.p.A. beim DSMZ am 14.11.2008, und Mischungen davon, wenn das menschliche Subjekt ein männliches Subjekt ist;
oder, wenn das menschliche Subjekt ein weibliches Subjekt ist, wird der mindestens eine Stamm und/oder Derivate davon aus der Gruppe ausgewählt, die umfasst oder alternativ besteht aus:
*Bifidobacterium lactis* BS01 LMG P-21384, hinterlegt von Anidral S.r.l. beim BCCM LMG am 31.01.2002, eine Mischung DLBL5S, vorzugsweise in einem Verhältnis 1:1:1:1:1, bestehend aus *Bifidobacterium longum* DLBL 07 DSM 25669, hinterlegt von Probiotical S.p.A. beim DSMZ am 16.02.2012, *Bifidobacterium longum* DLBL 08 DSM 25670, hinterlegt von Probiotical S.p.A. beim DSMZ am 16.02.2012, *Bifidobacterium longum* DLBL 09 DSM 25671, hinterlegt von Probiotical S.p.A. beim DSMZ am 16.02.2012, *Bifidobacterium longum* DLBL 10 DSM 25672, hinterlegt von Probiotical S.p.A. beim DSMZ am 16.02.2012, und *Bifidobacterium longum* DLBL 11 DSM 25673, hinterlegt von Probiotical S.p.A. beim DSMZ am 16.02.2012, und Mischungen davon.

3. Zusammensetzung zur Verwendung nach Anspruch 1 oder 2, wobei die Mischung ferner *Lactobacillus salivarius* LS01 DSM 22775 umfasst.

4. Zusammensetzung zur Verwendung nach mindestens einem der vorhergehenden Ansprüche, wobei die Verabreichung oral erfolgt.

5. Zusammensetzung zur Verwendung nach einem der vorhergehenden Ansprüche, wobei das Behandlungsverfahren die Verabreichung der Zusammensetzung an ein menschliches Subjekt umfasst, das von Morbus Parkinson betroffen oder gefährdet ist, an Morbus Parkinson zu erkranken.

## Revendications

1. Composition comprenant une quantité efficace d'un mélange qui comprend ou, alternativement, est constitué d'au moins une souche bactérienne appartenant à l'espèce *Lactobacillus salivarius,* dans laquelle ladite au moins une souche bactérienne, appartenant à l'espèce *Lactobacillus salivarius,* est *Lactobacillus salivarius* LS03 DSM 22776 ; ladite composition étant destinée à être utilisée dans un procédé de traitement curatif et/ou préventif d'au moins une pathologie neurodégénérative, ou d'au moins un symptôme ou trouble associé à celle-ci, chez un sujet humain, dans laquelle ledit procédé de traitement comprend l'administration de ladite composition à un sujet humain et dans laquelle ladite au moins une pathologie neurodégénérative est la maladie de Parkinson.

2. Composition destinée à être utilisée selon la revendication 1, dans laquelle ledit mélange comprend en outre au moins une souche bactérienne, et/ou des dérivés de celle-ci, choisis dans le groupe comprenant ou, alternativement, constitué de :
*Lactobacillus plantarum* LP01 LMG P-21021 déposé par Mofin Srl auprès de BCCM-LMG le 16/10/2001, *Lactobacillus reuteri* DLLRE 09 DSM 25685 déposé par Probiotical S.p.A. auprès de DSMZ le 16/02/2012, *Lactobacillus acidophilus* LA 02 DSM 21717 déposé par Probiotical S.p.A. auprès de DSMZ le 06/08/2008, *Lactobacillus rhamnosus* LR 06 DSM 21981 déposé par Probiotical S.p.A. auprès de DSMZ le 14/11/2008 et leurs mélanges, si le sujet humain est un sujet masculin ;
ou, si le sujet humain est un sujet féminin, ladite au moins une souche et/ou les dérivés de celle-ci sont choisis dans le groupe comprenant ou, alternativement, constitué de :
*Bifidobacterium lactis* BS01 LMG P-21384 déposée par Anidral S.r.l. auprès de BCCM LMG le 31/01/2002, un mélange DLBL5S, de préférence dans un rapport de 1:1:1:1:1, constitué de *Bifidobacterium longum* DLBL 07 DSM 25669 déposé par Probiotical S.p.A. auprès de DSMZ le 16/02/2012, *Bifidobacterium longum* DLBL 08 DSM 25670 déposé par Probiotical S.p.A. auprès de DSMZ le 16/02/2012, *Bifidobacterium longum* DLBL 09 DSM 25671 déposé par Probiotical S.p.A. auprès de DSMZ le 16/02/2012, *Bifidobacterium longum* DLBL 10 DSM 25672 déposé par Probiotical S.p.A. auprès de DSMZ le 16/02/2012 et *Bifidobacterium longum* DLBL 11 DSM 25673 déposé par Probiotical S.p.A. auprès de DSMZ le 16/02/2012, et leurs mélanges.

3. Composition destinée à être utilisée selon la revendication 1 ou 2, dans laquelle ledit mélange comprend en outre *Lactobacillus salivarius* LS01 DSM 22775.

4. Composition destinée à être utilisée selon au moins l'une des revendications précédentes, dans laquelle ladite administration a lieu par voie orale.

5. Composition destinée à être utilisée selon l'une quelconque des revendications précédentes, dans laquelle ledit procédé de traitement comprend l'administration de ladite composition à un sujet humain atteint de la maladie de Parkinson ou présentant un risque de contracter la maladie de Parkinson.
